# EUROPÄISCHE PATENTANMELDUNG

(11) **EP 3 069 759 A1**
(43) Veröffentlichungstag der Anmeldung: **21.09.2016**
(21) Anmeldenummer: 16158355.4
(22) Anmeldetag: 03.03.2016
(51) Int. Cl.: A61N 1/375, H01B 17/30, H01B 17/26

(54) **IMPLANTIERBARES ELEKTROMEDIZINISCHES GERÄT MIT EINER DURCHFÜHRUNG MIT EINEM EINSTÜCKIGEN KUNSTSTOFF-GRUNDKÖRPER**

(30) Priorität: 20.03.2015 US 201562135714 P
(71) Anmelder: BIOTRONIK SE & Co. KG, 12359 Berlin (DE)
(72) Erfinder: Sontheimer, Thomas, 90574 Roßtal (DE); Teske, Josef, 96103 Hallstadt (DE); Arnold, Michael, 91056 Erlangen (DE)
(74) Vertreter: Galander, Marcus

(57) **Zusammenfassung**

Implantierbares elektromedizinisches Gerät (1), umfassend ein Gerätegehäuse (3), in dem elektronische und elektrische Funktionseinheiten (7) untergebracht sind,
einen Gerätekopf (5) mit mindestens einer Elektrode oder einem Leitungsanschluss und eine zwischen Gerätegehäuse und Gerätekopf angeordnete Durchführung (11) für mindestens ein die Elektroden oder den Leitungsanschluss mit einer Funktionseinheit verbindendes elektrisches Leiterelement (13), wobei die Durchführung einen einstückigen Kunststoff-Grundkörper (15) umfasst. Der Kunststoff-Grundkörper (15) ist durch Ausspritzen eines umgebenden separaten Durchführungs-Flansches (17) und Umspritzen mindestens eines Anschlussstiftes (13), insbesondere mehrerer Anschlussstifte, gebildet. Am Gerätegehäuse (3) und/oder dem separaten Durchführungs-Flansch (17) und/oder mindestens einem elektrischen Leiterelement (13) ist mindestens ein sich in den Kunststoff-Grundkörper hinein erstreckender Fortsatz (13a, 17a) zur Verlängerung eines sich von der gehäuseäußeren Oberfläche (15a) des Kunststoff-Grundkörpers zu dessen gehäuseinnerer Oberfläche (15b) erstreckenden Diffusionsweges vorgesehen.

## Beschreibung

Die Erfindung betrifft ein implantierbares elektromedizinisches Gerät, umfassend ein Gerätegehäuse, in dem elektronische und elektrische Funktionseinheiten untergebracht sind, einen Gerätekopf mit mindestens einer Elektrode oder einem Leitungsanschluss und eine zwischen Gerätegehäuse und Gerätekopf angeordnete Durchführung für mindestens ein die Elektroden oder den Leitungsanschluss mit einer Funktionseinheit verbindendes elektrisches Leiterelement.

Derartige Geräte sind insbesondere als Herzschrittmacher oder implantierbare Kardioverter (speziell Defibrillatoren) seit langem in massenhaftem Einsatz. Es kann sich hierbei aber auch um eine weniger komplexe Vorrichtung, wie etwa eine Elektroden- oder Sensorleitung oder auch ein Cochlea-Implantat, handeln.

Die meisten praktisch bedeutsamen implantierbaren elektromedizinischen Geräte sind dazu vorgesehen, über geeignet platzierte Elektroden elektrische Impulse an reizbares Körpergewebe abzugeben. Um diese Funktion auszuführen, sind im Gehäuse des Gerätes elektronische/elektrische Funktionseinheiten zur Erzeugung der Impulse und zur geeigneten Steuerung der Impulserzeugung untergebracht, und außen am Gerät sind unmittelbar Elektroden oder Anschlüsse für mindestens eine Elektrodenleitung vorgesehen, in deren distalem Endabschnitt die Elektroden zur Impulsübertragung an das Gewebe angebracht sind. Die elektronischen/elektrischen Funktionseinheiten im Geräteinneren sind in einer Weise mit den äußeren Elektroden oder Elektrodenleitungsanschlüssen zu verbinden, die unter den speziellen Bedingungen des implantierten Zustandes eine absolut und dauerhaft verlässliche Funktion gewährleistet.

Bekannt sind insbesondere Durchführungen, deren Grund- und Isolationskörper im Wesentlichen aus Keramik oder Glas besteht, wobei auch mehrschichtige bzw. mehrteilige Aufbauten unter Einsatz von Metallen oder Metalloxiden entwickelt wurden und eingesetzt werden. Derartige bekannte Durchführungen erfüllen weitgehend die an sie gestellten Anforderungen. Jedoch müssen bei der Materialauswahl für die Komponenten Isolationskeramik/ Glas, Metall- oder Glaslot, Metallpin und Metallflansch die thermischen Ausdehnungskoeffizienten berücksichtigt werden, um eine über die vorgesehene Lebensdauer ausreichende Dichtheit gewährleisten zu können.

Beim konventionellen Design (Metallflansch - Lot - Isolationskeramik - Lot - Metallpin) kommt die Wirkung von nicht angepassten thermischen Ausdehnungskoeffizienten in erster Linie beim Abkühlen von Löttemperatur und Einschweißen der Durchführung ins Gehäuse zum Tragen. Resultieren können mechanische Zugspannungen, welche zur Materialtrennung und folglich zu etwaigen Leckagen der Durchführung führen können. Die bei konventionellen Durchführungen verwendeten keramischen und metallischen Komponenten sind durch den Lotwerkstoff miteinander verbunden; bei ungleichmäßiger Ausdehnung/Schrumpfung der Komponenten untereinander, einschließlich des Lotes, aufgrund von Aufheiz-/Abkühlvorgängen erzeugen die resultierenden relativen Längenänderungen entsprechende mechanische Spannungen, bis die Festigkeitswerte der verwendeten Materialien einer weiteren elastischen Spannungszunahme entgegenstehen. Duktile Komponenten/Werkstoffe (z.B. ein Flansch aus Titan oder ein Goldlot) erreichen ihre Streckgrenze und wandeln eine weitere Längenänderung in eine plastische Verformung bei moderater Spannungszunahme um. Spröde Komponenten/Werkstoffe (z.B. ein Isolator aus A1203 Keramik), aber auch bei der Verlötung entstehende Sprödphasen (z.B. zwischen Goldlot und Titan) hingegen erreichen ihre Zugfestigkeit durch das frühe Auftreten einer Werkstofftrennung, was Rissbildung und evtl. eine Leckage der Durchführung nach sich zieht.

Des Weiteren handelt es sich bei den konventionell verwendeten Materialien um sehr kostenintensive Materialien, die dann wiederum sehr kostenintensive Verbindungsprozesse, wie Beschichten und Hochtemperaturlöten benötigen.

Aus der EP 2 232 646 B1 ist eine hermetisch dichte Durchführungs-Struktur bekannt, die einen mehrteiligen Grund- bzw. Isolationskörper in Kombination mit abdichtenden (nicht strukturtragenden) Polymerschichten umfasst. Die Herstellung einer solchen Durchführung ist höchst aufwändig hinsichtlich der erforderlichen Arbeits- und Prüfschritte und auch hinsichtlich der Vorfertigung, Lagerhaltung und Zuführung vieler verschiedener Teile.

Auch die US 7,064,270 B2 beschreibt eine mehrteilig aufgebaute Durchführung, die speziell für eine Elektrodenleitung entwickelt wurde und mehrere aus Kunststoff gefertigte oder mit einer Kunststoffbeschichtung versehene Komponenten umfassen kann.

Aus der EP 2 388 044 A1 ist ein elektronisches Gerät bekannt, welches eine grundsätzlich einfach aufgebaute Durchführung aus einem flüssigkristallinen Polymeren hat. Einzelheiten der Gerätekonstruktion werden in dieser Druckschrift nicht offenbart.

Der Erfindung liegt die Aufgabe zugrunde, ein verbessertes implantierbares elektromedizinisches Gerät bereitzustellen, welches kostengünstig herstellbar und hochgradig zuverlässig ist.

Diese Aufgabe wird durch ein Gerät mit den Merkmalen des Anspruchs 1 gelöst. Zweckmäßige Fortbildungen des Erfindungsgedankens sind Gegenstand der abhängigen Ansprüche.

Die Erfindung geht von der Überlegung aus, eine Durchführung zu entwickeln, bei der als Verbindungskomponente zwischen den metallischen Leitern und dem Flansch ein Material zur Anwendung kommt, welches die unterschiedlichen thermischen Ausdehnungskoeffizienten der eingesetzten Komponenten toleriert, eine geringe Gas- und Flüssigkeitsdiffusion zwischen der einen und der anderen Seite der Durchführung besitzt und darüber hinaus auch eine kostengünstige Herstellung ermöglicht. Sie schließt den Gedanken ein, hierfür einen einstückigen Kunststoff-Grundkörper vorzusehen. Dieser übernimmt sowohl die Funktion des tragenden Teils und des Zusammenhaltens zwischen den Leiterelementen bzw. dem Leiterelement oder den Leiterelementen und einem umgebenden Durchführungs-Flansch, als auch die Funktion, diese gegeneinander zuverlässig elektrisch zu isolieren.

Von besonderem Vorteil sind der extrem einfache Aufbau und die leichte und kostengünstige Herstellung sowie auch die geringen Materialkosten.

In der Designphase ist es damit nicht mehr nötig, eine Abstimmung der zum Einsatz kommen-den Materialien hinsichtlich ihrer thermischen Wärmeausdehnungskoeffizienten vorzunehmen. Damit wird ermöglicht, weiterführende Designs/Materialien anzuwenden bzw. einzuführen, die Freiheitsgrade für die Gestaltung des Implantatgehäuses werden erhöht.

Die Verwendung eines Polymers für den Durchführungs-Grundkörper und zugleich als Isolator bringt im Übrigen folgende Vorteile:
- Das Einbringen und das Aushärten eines Duroplast-Werkstoffes kann bei Raumtemperatur vollzogen werden, so dass eine thermische Belastung, wie sie beim Löten einer konventionellen Durchführung entsteht, nicht auftritt.
- Thermische Spannungen, die beim Einbringen und Abkühlen einer heißen Polymerschmelze aus einem Thermoplastwerkstoff sowie beim Einschweißen des Flansches in ein Gehäuse entstehen, sind erwartungsgemäß geringer als bei konventionell hergestellten Durchführungen, da der Polymerwerkstoff einen wesentlich geringeren E-Modul als metallische Werkstoffe aufweist.

Darüber hinaus werden völlig neue Kosteneinsparpotentiale ermöglicht, ist doch der Spritzguss das Verfahren zur Herstellung von Bauteilen in hoher Geschwindigkeit, hohen Stückzahlen und geringen Kosten.

Besonders einfach und kostengünstig gestaltet sich die Herstellung in einer Ausführung des Kunststoff-Grundkörpers als Spritzgussteil. In einer Ausgestaltung dieser Ausführung ist der Kunststoff-Grundkörper durch Ausspritzen eines umgebenden separaten Durchführungs-Flansches und Umspritzen mindestens eines Anschlussstiftes, insbesondere mehrerer Anschlussstifte, gebildet.

In weiteren Ausführungen hat der Kunststoff-Grundkörper eine Füllung mit nicht organischen und nicht metallischen Teilchen, insbesondere Glas- und/oder Keramikteilchen. Insbesondere können die Teilchen zur Füllung des Kunststoff-Grundkörpers eine mittlere Teilchengröße von weniger als 20 µm, insbesondere von weniger als 10 µm, haben. Zur Erfüllung spezieller Anforderungen können auch andere Teilchengrößen in Betracht kommen, und der Grad der Füllung des Kunststoffs mit dem Zuschlagstoff wird im Hinblick auf die speziellen physikalischen Anforderungen und relevanten Eigenschaften des eingesetzten Kunststoffs und Zuschlagstoffs eingestellt.

In praktisch relevanten Ausführungen ist der Kunststoff-Grundkörper mit einem thermo- oder duroplastischen Kunststoff gebildet, insbesondere einem Epoxidharz, Polysulfon, PEEK oder einem flüssigkristallinen Polymer. Auch andere Kunststoffe als die hier genannten können in Betracht kommen.

In weiteren Ausführungen sind am Gerätegehäuse und/oder dem separaten Durchführungs-Flansch und/oder mindestens einem elektrischen Leiterelement mindestens ein sich in den Kunststoff-Grundkörper hinein erstreckender Fortsatz zur Verlängerung eines sich von der gehäuseäußeren Oberfläche des Kunststoff-Grundkörpers zu dessen gehäuseinnerer Oberfläche erstreckenden Diffusionsweges vorgesehen. In einer speziellen Ausgestaltung weist der separate Durchführungs-Flansch mehrere sich im Wesentlichen senkrecht zu seiner Umfangsfläche erstreckende Fortsätze und/oder der oder jeder eingespritzte Anschlussstift mindestens einen sich senkrecht zu seinem Längsverlauf erstreckenden scheibenförmigen Fortsatz auf. Es versteht sich, dass die Fortsätze an den einzelnen Teilen relativ zueinander derart angeordnet sind, dass sie sich nicht gegenseitig berühren, sondern für eine wirksame elektrische Isolation hinreichende Abstände zueinander haben.

In einer weiteren Ausführung weist der separate Durchführungs-Flansch einen eingefügten Masseanschluss auf.

In einer weiteren Ausführung ist der separate Durchführungs-Flansch als durch Metall-Pulverspritzgießen gebildetes Seitenteil mit eingespritztem Masseanschluss ausgeführt. Es handelt sich hierbei um ein etabliertes Verfahren zur kostengünstigen Herstellung hochwertiger Metallteile, welches dem Fachmann an sich vertraut ist und daher nicht genauer beschrieben werden muss. Als eingesetztes Metall kommt für die Ausführung der Erfindung insbesondere Titan oder eine Titanlegierung in Betracht; grundsätzlich können aber auch verwandte Metalle, wie Niob, Molybdän, Tantal, Wolfram, Vanadium, Zirkon oder Iridium und Legierungen derselben oder Nickel oder Palladium oder deren Legierungen oder Stähle, insbesondere medizinische Stähle wie z. B. 316L zum Einsatz kommen.

In weiteren Ausführungen der Erfindung ist auf der gehäuseäußeren und/oder der gehäuseinneren Oberfläche des Kunststoff-Grundkörpers eine oder mehrere sich über die jeweilige gesamte Oberfläche und bevorzugt auch den angrenzenden Bereich des Innenumfangs des Durchführungs-Flansches erstreckende, insbesondere biokompatible, Barriereschicht(en) aufgebracht. Eine solche Barriere aus einer oder mehreren Dünnschichten verbessert die Dichtigkeit der Durchführung des Geräts insbesondere hinsichtlich der Gas- und Flüssigkeitsdiffusion aus umgebender Körperflüssigkeit in das Gerät. Auf die Biokompatibilität der Beschichtung(en) kommt es primär bei einer geräte-äußeren Anwendung an; beim Vorsehen einer beidseitigen Beschichtung ist es indes von Vorteil, diese aus dem gleichen Schichtmaterial zu bilden.

In einer möglichen Ausgestaltung der vorgenannten Ausführung ist die Barriereschicht/ sind die Barriereschichten als durch Vakuumbeschichtung aufgebrachte Dünnschicht(en) ausgebildet. Insbesondere weist die Barriereschicht bzw. das Schichtsystem eine Metalloxidschicht, insbesondere Titanoxid, Aluminiumoxid, Siliziumoxid, Nioboxid oder ähnliche, auf. Die Erzeugung derartiger Metalloxidschichten ist mit bekannten Beschichtungsverfahren und kostengünstig kommerziell verfügbaren Targets leicht möglich und dem Fachmann vertraut, so dass es hinsichtlich dieser Ausgestaltung, hier keiner weiteren Beschreibung bedarf.

Die Leiterelemente der Implantatdurchführung können z. B. aus den Elementen Pt, Ir, Nb, Ta, Ti, Fe, Cr, Ni oder Legierungen daraus bestehen.

In einer weiteren Ausführungsform können die Leiterlemente zweiteilig sein, z. B. auf der Implantatinnenseite jeweils einen weichlötbaren Bereich besitzen, z. B. aus Cu, Ni, Au, Ag oder Legierungen daraus, und auf der Implantataußenseite einen schweißbaren, biokompatiblen Bereich, z. B. aus Ti, Nb, Ta, Fe, Ni, Cr, oder Legierungen daraus. Diese Ausführungsform hat den Vorteil, dass sie in einem sog. Reflow-Lötprozess an ein Elektronikmodul wie jedes andere oberflächenmontierbare (SMT) elektronische Bauteil auch elektrisch und mechanisch fest angebunden werden kann, ohne dass Bohrungen für die Anschlussstifte im Elektronikmodul vorgesehen sein müssen. Vorteilhafterweise können diese zweiteiligen Stifte kostengünstig in folgender Prozessfolge hergestellt sein: Walzen, Stanzen, Umformen mit eventuellen Zwischen-Glühschritten. Auch das Masse-Leiterelement kann auf diese Weise hergestellt sein, nicht nur die anderen Leiterlemente, die für die Signalübertragung verantwortlich sind.

In einer weiteren Ausführungsform kann direkt ein Hochfrequenzfilter an die Signal übertragenden Leitelemente angefügt sein, z. B. durch Weichlöten, Schweißen, leitfähiges Kleben oder Klemmen. Dieses Hochfrequenzfilter ist als Tiefpass oder als Bandsperre ausgeführt und hat die Aufgabe, mögliche hochfrequente Störstrahlung aus dem Implantatgehäuse-Inneren abzuhalten. In einer weiteren, vorteilhaften Ausführung kann das Hochfrequenzfilter direkt vom Kunststoffkörper der Durchführung mechanisch fest eingegossen sein.

Vorteile und Zweckmäßigkeiten der Erfindung ergeben sich im Übrigen aus der Beschreibung von Ausführungsbeispielen anhand der Figuren. Von diesen zeigen:
- Fig. 1: eine schematische, teilweise geschnittene Darstellung eines implantierbaren elektromedizinischen Geräts,
- Fig. 2: eine schematische Querschnittsdarstellung (Teilansicht) eines Ausführungsbeispiels der Erfindung und
- Fig. 3: eine schematische Querschnittsdarstellung (Teilansicht) eines weiteren Ausführungsbeispiels der Erfindung.

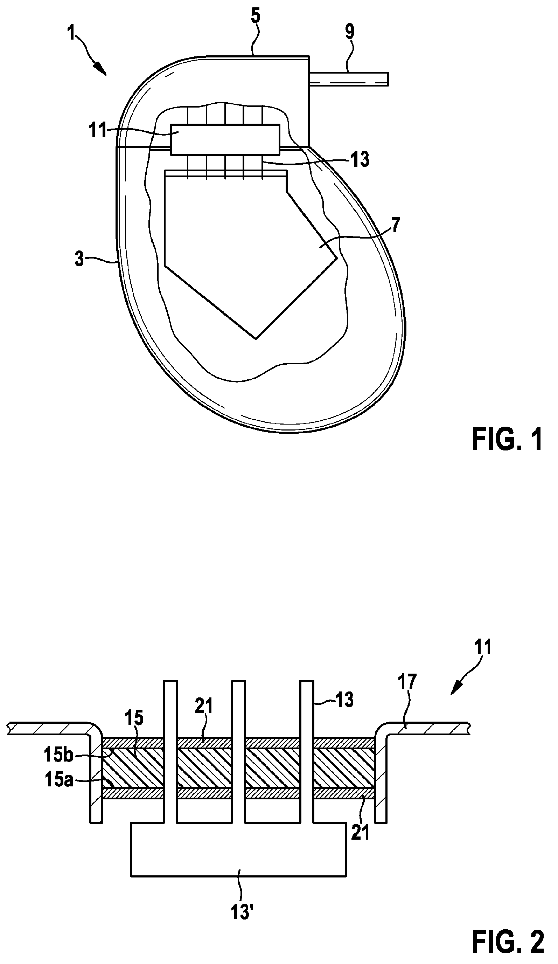

Fig. 1 zeigt einen Herzschrittmacher 1 mit einem Schrittmachergehäuse 3 und einem Kopfteil (Header) 5, in dessen Innerem neben anderen elektronischen Komponenten eine Leiterplatte (PCB) 7 angeordnet und mit dessen im Header angeordneten (nicht gezeigten) Leitungsanschluss eine Elektrodenleitung 9 verbunden ist. Eine zwischen Gerätegehäuse 3 und Header 5 vorgesehene Durchführung 11 umfasst eine Mehrzahl von Anschlussstiften 13. Die Anschlussstifte sind an einem Ende durch eine entsprechende Bohrung in der Leiterplatte gesteckt und mit dieser weich verlötet.

Fig. 2 zeigt schematisch in Art einer Querschnittsdarstellung eine erste Ausführung der Durchführung 11, und zwar mit einem spritzgegossenen Kunststoff-Grundkörper 15 in einem kalt umgeformten Durchführungs-Flansch 17, wobei der Kunststoff-Grundkörper 15 sowohl auf der geräteäußeren Oberfläche 15a als auch auf der geräteinneren Oberfläche 15b mit einem diffusionshemmenden Barriereschichtsystem 21 (gebildet beispielsweise durch Sputtern oder Vakuumbedampfung) versehen ist. In den Kunststoff-Grundkörper 15 sind Anschlussstifte 13 als Leiterelemente eingespritzt. In der Darstellung ist ein gestanzter Metallkamm 13' zu sehen, der zur Fixierung der Leiterelemente in der Spritzgussform dient und bei Fertigstellung der Durchführung entfernt wird. Er kann beispielsweise mit Au galvanisiert sein, womit also Au-beschichtete und somit weichlötbare Anschlussstifte bereitgestellt werden.

In weiteren Ausführungen (ohne Zeichnung) ist der Kunststoffgrundkörper 15 jeweils nur auf einer Seite - entweder auf der geräteinneren oder auf der geräteäußeren Seite - mit einem diffusionshemmenden Barriereschichtsystem 21 versehen.

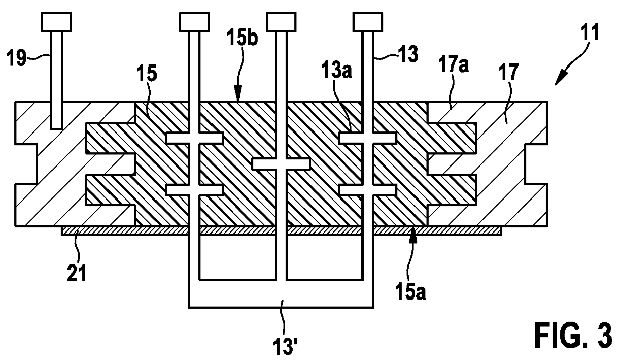

Fig. 3 zeigt, in einer an Fig. 2 angelehnten Darstellung und unter Verwendung der gleichen Bezugsziffern für funktionsgleiche Teile, eine modifizierte Ausführung der Durchführung 11. Bei dieser ist anstelle eines kalt umgeformten Flansches ein durch Metall-Pulverspritzgießen (MiM-Technologie) vorgeformter Flansch 17 vorgesehen, der an seinem Innenumfang mehrere nach innen in das Material des Kunststoff-Grundkörpers 15 ragende ringförmige Fortsätze 17a trägt. Korrespondierend hierzu sind bei der Ausführung nach Fig. 3 die Anschlussstifte 13 mit einer oder zwei Fixierungsscheiben 13a versehen, die sich ebenso wie die ringförmigen Fortsätze 17a mit dem Polymermaterial des Grundkörpers 15 verzahnen und zugleich in einer Weise versetzt in diesen hineinragen, dass ein verlängerter Diffusionsweg zwischen der geräteäußeren Oberfläche 15a und der geräteinneren Oberfläche 15b geschaffen wird. Zur zusätzlichen Verbesserung der Diffusionsfestigkeit gegenüber gasförmigen oder flüssigen Bestandteilen von Körperflüssigkeit, die im Gebrauchszustand das elektromedizinische Gerät umgibt, ist hier auf der geräteäußeren Seite wiederum ein Barriereschichtsystem 21 aufgebracht.

Die Ausführung der Erfindung ist auch in einer Vielzahl von Abwandlungen der hier gezeigten Beispiele und weiter oben hervorgehobenen Aspekte der Erfindung möglich. So kann insbesondere die geometrische Gestalt des Durchführungs-Flansches (sofern ein solcher überhaupt separat vorgesehen ist) und der Leiterelemente (Anschlussstifte) vielgestaltig modifiziert sein, und es können auf andere Weise vorgefertigte Flansche und/oder Anschlussstifte in den Kunststoff-Grundkörper der Durchführung eingebettet sein. Ein Diffusions-Barriereschichtsystem aus einer oder mehreren Dünnschichten kann auf beiden Oberflächen des Grundkörpers vorgesehen sein oder, je nach Diffusionseigenschaften des eingesetzten Kunststoffs und der gegebenenfalls vorgesehenen Füllung, auch ganz entfallen.

## Patentansprüche

1. Implantierbares elektromedizinisches Gerät (1), umfassend
ein Gerätegehäuse (3), in dem elektronische und elektrische Funktionseinheiten (7) untergebracht sind,
einen Gerätekopf (5) mit mindestens einer Elektrode oder einem Leitungsanschluss und
eine zwischen Gerätegehäuse und Gerätekopf angeordnete Durchführung (11) für mindestens ein die Elektroden oder den Leitungsanschluss mit einer Funktionseinheit verbindendes elektrisches Leiterelement (13),
wobei die Durchführung einen einstückigen Kunststoff-Grundkörper (15) umfasst.

2. Gerät nach Anspruch 1, wobei der Kunststoff-Grundkörper (15) als Spritzgussteil ausgebildet ist.

3. Gerät nach Anspruch 2, wobei der Kunststoff-Grundkörper (15) durch Ausspritzen eines umgebenden separaten Durchführungs-Flansches (17) und Umspritzen mindestens eines Anschlussstiftes (13), insbesondere mehrerer Anschlussstifte, gebildet ist.

4. Gerät nach einem der vorangehenden Ansprüche, wobei der Kunststoff-Grundkörper (15) eine Füllung mit nicht organischen und nicht metallischen Teilchen, insbesondere Glas- und/oder Keramikteilchen aufweist.

5. Gerät nach Anspruch 4, wobei die Teilchen zur Füllung des Kunststoff-Grundkörpers (15) eine mittlere Teilchengröße von weniger als 20 µm, insbesondere von weniger als 10 µm, haben.

6. Gerät nach einem der vorangehenden Ansprüche, wobei der Kunststoff-Grundkörper (15) mit einem thermo- oder duroplastischen Kunststoff gebildet ist, insbesondere einem Epoxidharz, Polysulfon, PEEK oder einem flüssigkristallinen Polymer.

7. Gerät nach einem der vorangehenden Ansprüche, wobei am Gerätegehäuse (3) und/oder dem separaten Durchführungs-Flansch (17) und/oder mindestens einem elektrischen Leiterelement (13) mindestens ein sich in den Kunststoff-Grundkörper hinein erstreckender Fortsatz (13a, 17a) zur Verlängerung eines sich von der gehäuseäußeren Oberfläche (15a) des Kunststoff-Grundkörpers zu dessen gehäuseinnerer Oberfläche (15b) erstreckenden Diffusionsweges vorgesehen ist.

8. Gerät nach Anspruch 7, wobei der separate Durchführungs-Flansch (17) mehrere sich im Wesentlichen senkrecht zu seiner Umfangsfläche erstreckende Fortsätze (17a) und/oder der oder jeder eingespritzte Anschlussstift (13) mindestens einen sich senkrecht zu seinem Längsverlauf erstreckenden scheibenförmigen Fortsatz (13a) aufweist.

9. Gerät nach einem der Ansprüche 3 bis 8, wobei der separate Durchführungs-Flansch (17) einen eingefügten Masseanschluss (19) aufweist.

10. Gerät nach einem der Ansprüche 3 bis 9, wobei der separate Durchführungs-Flansch (17) als durch Metall-Pulverspritzgießen gebildetes Seitenteil mit eingespritztem Masseanschluss (19) ausgeführt ist.

11. Gerät nach einem der Ansprüche 3 bis 10, wobei auf der gehäuseäußeren (15a) und/oder der gehäuseinneren Oberfläche (15b) des Kunststoff-Grundkörpers (15) eine sich über die jeweilige gesamte Oberfläche und insbesondere auch den angrenzenden Bereich des Innenumfangs des Durchführungs-Flansches erstreckende, insbesondere biokompatible, Barriereschicht (21) aufgebracht ist.

12. Gerät nach Anspruch 11, wobei ein Barriereschichtsystem (21) mit einer oder mehreren durch Vakuumbeschichtung(en) aufgebrachten Dünnschicht(en) vorgesehen ist.

13. Gerät nach Anspruch 11 oder 12, wobei die Barriereschicht oder das Barriereschichtsystem (21) mindestens eine Metalloxidschicht, insbesondere Titanoxid, Aluminiumoxid, Siliziumoxid, Nioboxid oder ähnliche, aufweist.

14. Gerät nach einem der vorangehenden Ansprüche, wobei das Leiterelement (13) als im Wesentlichen zylindrischer Anschlussstift ausgebildet ist, der insbesondere ein nagelkopf-artig verformtes Ende aufweist.

15. Gerät nach einem der vorangehenden Ansprüche, ausgebildet als Elektrostimulationsgerät, insbesondere Herzschrittmacher oder Kardioverter, wobei der Leitungsanschluss am Gerätekopf zum Anschluss einer Elektrodenleitung ausgebildet ist.
